# EUROPÄISCHE PATENTANMELDUNG

(11) **EP 0 529 375 A1**
(43) Veröffentlichungstag der Anmeldung: **03.03.1993**
(21) Anmeldenummer: 92113533.1
(22) Anmeldetag: 08.08.1992
(51) Int. Cl.: A61M 3/02

(54) **Irrigator**

(30) Priorität: 28.08.1991 DE 4128420
(71) Anmelder: PARDES SPEZIALSTRÜMPFE GmbH & Co. KG, D-63477 Maintal (DE)
(72) Erfinder: Barth, Reinhart, c/o Pardes Spezialstrümpfe GmbH &, W-6457 Maintal 1 (DE)
(74) Vertreter: Baronetzky, Klaus, Dipl.-Ing.

(57) **Zusammenfassung**

Die Erfindung betrifft einen Irrigator (10), bei dem ein Beutel (12) in einer Spülflüssigkeitskammer (14) mit Spülflüssigkeit gefüllt werden kann. Die Spülflüssigkeitskammer (14) ist verschließbar und über eine Membran (18) von einer Luftkammer (16) getrennt. Die Luftkammer (16) ist über eine Pumpe (36) unter Druck setzbar.

## Beschreibung

Die Erfindung betrifft einen Irrigator gemäß dem Oberbegriff von Anspruch 1.

Ein derartiger Irrigator wird von Kolostomieträgern verwendet, um eine Darmspülung oder Irrigation durchzuführen. Durch eine sorgfältige Irrigation läßt sich eine ausscheidungsfreie Periode von beispielsweise 48 Stunden erzielen, wobei die je patientenspezifische Dauer der ausscheidungsfreien Zeit stark von der Restdarmlänge und weiteren Parametern abhängt.

Die bislang üblichen und verbreiteten Irrigatoren bestehen im wesentlichen aus einem Beutel, der in seinem oberen Bereich mit mindestens einer Aufhängeöse versehen ist. In dem Beutel ist eine Art Trichter eingearbeitet, so daß die Befüllung mit Leitungswasser ohne weiteres möglich ist. An dem Beutel ist unten ein Ableitungsschlauch angeschlossen, der in einem Konus mündet und mit einer Klemme, wie beispielsweise eine Rollklemme gesperrt werden kann. Für die Darmspülung wird nun der Beutel mit Wasser gefüllt und an einem Wandhaken aufgehängt, so daß sich aufgrund des Höhenunterschieds im Beutel an dem Konus ein Druck ausbildet, der für die Darmspülung ausreicht.

Nachteilig bei diesem bekannten Irrigator ist die Notwendigkeit, daß stets eine in geeigneter Höhe angebrachte Aufhängevorrichtung vorhanden sein muß, was nicht immer gewährleistet ist, beispielsweise, wenn sich der Kolostomieträger auf Reisen befindet. Zwar sind die besseren Irrigatoren mit einem Rückschlagventil am Wassereinlaß versehen. Wenn der Beutel jedoch aus Versehen von der Aufhängevorrichtung herunterfallt, und etwas ungünstig gerade auf dem Wasserzufluß aufprallt, läßt sich ein Versagen dieses Rückschlagventils nicht ganz vermeiden, zumal es einerseits sehr leichtgängig sein muß, damit es durch das einfließende Wasser geöffnet wird, und andererseits an dem im Grunde sehr flexiblen Beutel eingearbeitet sein muß.

Zur Beseitigung der Nachteile der an sich sehr flexibel einsetzbaren Irrigatoren ist ferner ein elektrischer Irrigator bekannt geworden, der mit einem etwas abweichenden Prinzip arbeitet: anstelle des Beutels ist ein Wassertank vorgesehen. Ein stabiles Gehäuse nimmt den Wassertank, eine regelbare Förderpumpe und eine Elektronik sowie eine Stromversorgung auf. Mit der Elektronik ist die Leistung der Pumpe regelbar, wobei in der bekannten Ausgestaltung die Stromversorgung durch Akkumulatoren gebildet wird, die nach jedem Spülvorgang nachgeladen werden müssen.

Dieser elektrische Irrigator ist zwar im wesentlichen lageunabhängig, so daß eine Aufhängevorrichtung nicht erforderlich ist. Zudem kann die Absperrklemme für den Ableitungsschlauch entfallen. Das Gerät ist jedoch so schwer, daß es sich in der Praxis nicht durchgesetzt hat, denn insbesondere auf Reisen stellt eine zusätzliche Gewichtsbelastung eines Kolostomieträgers ohne Hilfsperson von beispielsweise mehr als einem Kilogramm eine nicht zumutbare Belastung dar. Zudem sind die Akkumulatoren des Geräts bereits nach einer einmaligen Irrigation erschöpft. Zwar kann mit einem zusätzlich mitzuführenden und somit ein zusätzliches Gewicht darstellenden Ladegerät der Akkumulatorensatz nachgeladen werden. Um das Nachladen durchführen zu können, ist der Kolostomieträger jedoch wiederum auch auf Reisen auf die Zurverfügungstellung einer entsprechenden Steckdose für beispielsweise 10 Stunden angewiesen. Aufgrund der an sich bekannten Eigenschaften von Akkumulatoren sollte zudem das Nachladen gleich nach der Irrigation einsetzen, was eine zusätzliche Bindung für den Kolostomieträger darstellt.

Ein reiner Batteriebetrieb mit mitzuführenden Ersatzbatterien kommt aus einer Vielzahl von anderen Gründen nicht in Betracht.

Demgegenüber liegt die Erfindung die Aufgabe zugrunde, einen Irrigator gemäß dem Oberbegriff von Anspruch 1 zu schaffen, der eine flexiblere Handhabung mit einer höheren Gebrauchsicherheit verbindet, ohne daß ins Gewicht fallende zusätzliche Kosten oder ein relevantes Zusatzgewicht erforderlich würde.

Diese Aufgabe wird erfindungsgemäß durch Anspruch 1 gelöst. Vorteilhafte Weiterbildungen ergeben sich aus den Unteransprüchen.

Der erfindungsgemäße Irrigator weist zunächst den Vorteil auf, daß er unabhängig von einer Aufhängvorrichtung einsetzbar ist. Dies bedeutet, daß eine Darmspülung auch an einer Toilette vorgenommen werden kann, die nicht mit einer entsprechenden Aufhängevorrichtung ausgerüstet ist. Das Gewicht des erfindungsgemäßen Irrigators entspricht jedoch praktisch dem des verbreiteten manuellen Irrigator, denn die zusätzlichen erforderlichen Teile sind leicht, und das vergleichsweise große Rückschlagventil wie auch der trichterförmig ausgebildete Wasserzufluß an dem Beutel kann dafür entfallen bzw. durch einen Zuleitungsschlauch ersetzt werden.

Besonders günstig ist es, daß die Tatsache ausnutzt werden kann, daß der bekannte Irrigatorbeutel bislang ohnehin eine gewisse Druckfestigkeit haben mußte, um nicht von sich aus aufzuplatzen, wenn der gefüllte Beutel einmal hinfällt. Diese Druckfestigkeit wird nun ausgenutzt, um über die erfindungsgemäße Membran, die lediglich den Beutel in zwei Kammern teilt, einen Druck aufzubauen, und mit diesem eine völlig Lageunabhängigkeit zu erzielen. Ein in wassergefährdeten Umgebungen stets besonders abzuschottendes elektrisches System läßt sich dennoch völlig vermeiden, indem beispielsweise einfach eine kleine Handpumpe eingesetzt wird, um den erforderlichen Druck bereitzustellen. Über die Membran in dem Beutel wirkt der in der Luftkammer aufgebauten Druck auch auf die Spülflüssigkeitskammer. Aufgrund der Elastizität des Luftvolumens wird zudem eine Druckreserve in der Weise bereitgestellt, das auch gegen Ende des Spülzyklusses ein ausreichender Druck zu Verfügung steht.

Besonders günstig in Zusammenhang hiermit ist der Einsatz eines Druckminderes, wobei ein Druckminderventil in an sich bekannter Weise als kleines und leichtbauendes Bauteil in den Ableitungsschlauch eingefügt sein kann. Das Druckminderventil kann hierbei beispielsweise einen fest eingestellten Maximaldruck von beispielsweise 250 mbar aufweisen, der in der Praxis auf einen Wert von beispielsweise 100 mbar reduziert wird. Während des gesamten Spülzyklus liegt dann der gewünschte Spüldruck an, wobei sich beispielsweise auch mit einer Hand-Ballpumpe ohne weiteres einen Druck von erheblich mehr als 250 mbar erzeugen läßt.

Indem die Spülflüssigkeitskammer verschließbar ist, kann die Luftkammer lageunabhängig aufgepumpt werden. Somit ergibt sich ein entscheidender Vorteil gegenüber den bekannten Irrigatoren bereits aus dem Verschluß des Beutels, während andererseits das Aufbauen des erforderlichen Drucks im Grunde mit beliebigen geeigneten manuellen Mitteln geschehen kann.

In diesem Zusammenhang ist die Verwendung einer Hand-Ballpumpe günstig, da sie einerseits die Handmuskulatur des Patienten eher kräftigt und so in der Tendenz zu dessen Fitneß beiträgt, andererseits für die Betätigung der Hand-Ballpumpe Bauchmuskeln des Kolostomieträgers nicht beansprucht werden.

Besonders günstig ist es, daß der erfindungsgemäße Irrigator von der Funktionalität her dem bekannten elektrischen Irrigator mindestens gleichkommt. Aufgrund des einfachen Aufbaus ist die Störanfälligkeit und auch die Reinigungsmöglichkeit erheblich verbessert.

Darüber hinaus ist der erfindungsgemäße Irrigator hinsichtlich des Packvolumens sehr günstig, denn der verwendete Beutel kann leicht zusammengefaltet werden, und die Hand-Ballpumpe ist ebenfalls elastisch. Damit sind die einzigen nicht-weichen Bauteile die Schläuche und der ebenfalls aus Kunststoff bestehende Druckminderer, der zudem sehr klein baut. Das Gewicht des erfindungsgemäßen Irrigators beträgt somit nur einen Bruchteil des elektrischen Irrigators, so daß sich erhebliche Handhabungsvorteile ergeben.

Besonders günstig lassen sich die Materialeigenschaften des verwendeten Kunststoffs wie Polyethylen, Polypropylen oder Polyvinylchlorid für die Funktion ausnutzen: die Wandstärke des verwendeten Beutels entspricht praktisch der Wandstärke des bekannten Irrigators, wobei die Luftkammer prall aufgepumpt sein kann, ohne das eine Überdehnung des Beutels stattfindet. Hier läßt sich ein leichter Puffereffekt sowohl des elastischen Luftvolumens als auch des leicht elastischen Beutelmaterials ausnutzen. Zudem kann der Einfachheit halber die Hand-Ballpumpe so ausgebildet sein, daß der mit ihr erzeugbare Druck - mit einer gewissen Sicherheitsreserve - nicht den Maximaldruck der Luftkammer übersteigt.

An sich ist ferner aus der DE-GM 83 03 620.2 ein.Gerät zur Infusion oder Transfusion von Körperflüssigkeiten bekannt, das mit einer Membran und einer Ballpumpe arbeitet. Dieses Gerät weist jedoch einen festen Tank auf, so daß der erwünschte Federungseffekt, der übrigens noch durch die Volumenvergrösserungsfalten gemäß Anspruch 9 unterstützt sein kann, hier nicht auftreten kann. Da kein Ableitungsschlauch im Sinne der Erfindung vorgesehen ist, sondern die Zufuhr wie auch das etwaige Ausdrücken von Blut über einen deckelseitigen Auslaß vorgesehen ist, ist es nicht verwunderlich, daß auch kein Druckminderer vorgesehen ist. Zudem ist dieses Gerät für die Transfusion bzw. Reinfusion von Blut oder Plasmaersatzstoffen ausgestaltet und kein Irrigator.

Besonders günstig kann erfindungsgemäß die Zuleitung und die Ableitung an dem gleichen Anschluß der Spülflüssigkeitskammer vorgesehen sein, wobei es sich anbietet, lediglich einen Durchbruch durch die Wandung des Beutels vorzusehen, und Zu- und Ableitung über ein T-Stück miteinander zu kombinieren. Diese Konstruktion bildet den Vorteil, daß lediglich ein Steifigkeitssprung Beutel-Schlauch besteht, so daß die Stellen hoher Materialbeanspruchung beim Zusammenfalten des Beutels gering bleiben können.

Gemäß einer weiteren Ausgestaltung der Erfindung ist es vorgesehen, den Zuleitungs- und den Ableitungsschlauch miteinander zu kombinieren. Hierzu wird der in dem Ableitungsschlauch vorgesehene Druckminderer über einen Bypass überbrückt, der bei der Ableitungsfunktion gesperrt ist und lediglich das Füllen des Beutels erlaubt, wenn der Regler sich in der Füllstellung befindet. Mit dieser Ausgestaltung läßt sich der Material- und Gewichtsaufwand weiter reduzieren.

Es versteht sich, daß die erfindungsgemäße Membran luft- und wasserdicht in den aus Kunststoff bestehenden Beutel eingeschweißt sein kann. Die Spülflüssigkeitskammer kann ein Maximalvolumen von beispielsweise 2 Litern aufweisen, so daß eine ausreichende Spülmenge für die Spülung "in einem Zug" zu Verfügung steht. Wenn das vorstehend erläuterte kombinierte Zuleitungs-/Ableitungsschlauchsystem nicht eingesetzt wird, kann die Zuleitung mittels eines einfachen Ventils verschlossen werden.

Besonders günstig ist es auch, wenn die Schlauchverbindung für den Beutel geschraubt oder gesteckt ist. Dies bietet die Möglichkeit einer vergleichsweise leichten Reinigung im Falle einer Verschmutzung.

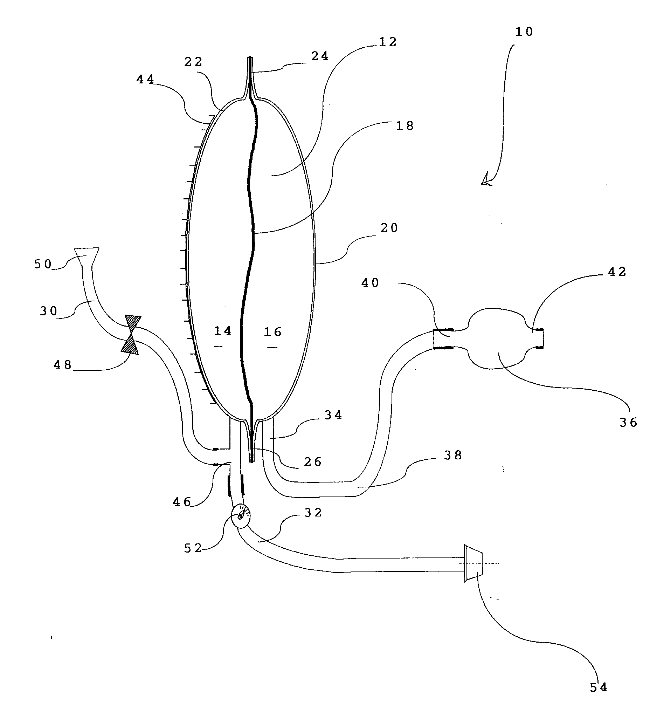

Weitere Vorteile, Einzelheiten und Merkmale ergeben sich aus der nachstehenden Beschreibung eines Ausführungsbeispiels der Erfindung anhand der Zeichnung.

Es zeigt:
Die einzige Figur eine Ausführungsform eines erfindungsgemäßen Irrigators in einer schematischen Ansicht.

Ein erfindungsgemäßer Irrigator 10 gemäß dem in der einzigen Figur dargestellten Ausführungsbeispiel weist einen Beutel 12 auf, der aus einer Spülflüssigkeitskammer 14 und einer Luftkammer 16 besteht. Die Spülflüssigkeitskammer 14 und die Luftkammer 16 sind über eine Membran 18 miteinander verbunden. Die Membran 18 besteht aus dem gleichen Material wie die Außenwände 20 und 22 des Beutels 12 und ist an Nähten, von denen zwei Nähte 24 und 26 in der Figur dargestellt sind, mit diesen ringsum verschweißt. Beim Verschweißen wird darauf geachtet, daß die Membran 18 im wesentlichen so bauchig gehalten ist wie die Außenwände 20 und 22. Der bauchige Schnitt sowohl der Membran als auch der beiden Außenwände 20 und 22 hat den Vorteil, daß für das Zusammenlegen diese drei Schichten paßgerecht aufeinandergedrückt werden können, so daß das Packvolumen extrem klein wird.

Seitlich gegeneinander versetzt, jedoch ebenfalls einander gegenüberliegend weisen die Kammern 14 und 16 je einen Anschluß auf. Ein Anschluß 28 der Spülflüssigkeitskammer 14 ist für die Verbindung mit einer schlauchförmigen Zuleitung 30 und einer Ableitung 32 vorgesehen. Ein Anschluß 34 der Luftkammer 16 ist für die Verbindung mit einer Pumpe 36 vorgesehen, die im Beispielsfalle als Hand-Ballpumpe ausgebildet ist. Die Pumpe 36 weist in an sich bekannter Weise an ihren beiden Enden je ein Rückschlagventil auf, so daß ein wiederholtes Zusammenpressen und Freigeben des Pumpvolumens der elastischen Pumpe 36 zu der erwünschten Pumpwirkung führt. Die so gepumpte Luft wird über einen Luftschlauch 38 und den Anschluß 34 in die Luftkammer 16 eingebracht.

In dem dargestellten Ausführungsbeispiel ist die Pumpe 36 über eine Steckverbindung 40 mit dem Luftschlauch 38 verbunden. Zur Entlüftung der Luftkammer 16, wenn beispielsweise der Irrigator 10 für Transportzwecke zusammengefaltet werden soll, oder die Spülflüssigkeitskammer 14 neu mit Wasser befüllt werden soll, wird einfach die Steckverbindung 40 gelöst, so daß die Restluft in der Luftkammer 16 entweichen kann. Durch leichten Druck auf die Luftkammer 16 kann hier gegebenenfalls manuell nachgeholfen werden.

Gemäß einer weiteren bevorzugten Ausgestaltung der Erfindung ist es vorgesehen, die Pumpe 36 an ihrem Lufteinlaß 42 mit einer gleichen Steckverbindung auszustatten. Durch einfaches Umdrehen und erneutes Einstecken der Pumpe 36 in die Steckverbindung 40 zum Luftschlauch 38 hin kann dann die Restluft aus der Luftkammer 16 herausgepumpt werden, was insbesondere für Reisezwecke sehr günstig ist.

Alternativ besteht die Möglichkeit, ein separates, in der Zeichnung nicht dargestelltes Ablaßventil irgendwo im Bereich der Luftkammer 16, des Schlauchs 38 und/oder der Pumpe 36 zu verwenden.

Ferner ist es günstig, wenn sich die Anschlüsse 34 und 28 im wesentlichen in der Verlängerung der Membran 18 nahe der Naht 26 erstrecken. Dies bietet den Vorteil, daß die Anschlüsse 28 und 34 beim Verpacken sich günstig in der Verlängerung des dann zusammengelegten Beutels 12 erstrecken, und somit nicht auftragen, zumal sie einander nicht seitlich überlappen.

Die Spülflüssigkeitskammer 14 ist im Bereich der Außenwand 22 mit einer Markierung 44 versehen, die den Füllungsgrad der Spülflüssigkeitskammer 14 bei vollständig entleerter Luftkammer 16 anzeigt. Es versteht sich, daß während des Befüllens der Spülflüssigkeitskammer 14 mit Wasser über die Zuleitung 30 die 5teckverbindung 40 aufgetrennt sein sollte, so daß die Restluft aus der Luftkammer 16 entweichen kann, wenn sie nicht bereits herausgepumpt worden ist.

Der Anschluß 28 ist über ein T-Stück 46 mit der Zuleitung 30 verbunden, die ihrerseits mit einem einfachen Sperrventil 48 ausgestattet ist. Zur Befüllung wird eine leicht konische Aufweitung 50 der Zuleitung 30 unter einen nicht dargestellten Wasserhahn gehalten, und die Spülflüssigeitskammer 14 so gefüllt. Etwaige, in der Spülflüssigkeitskammer 14 verbleibende Restluft stört hierbei nicht, solange sie nicht eine ausreichende Füllung der Spülflüssigkeitskammer 14 verhindert, und kann gewünschtenfalls durch ein Umdrehen des Beutels 12 über die Zuleitung 30 entweichen.

An das T-Stück 46 ist ferner die Ableitung 32 angeschlossen, die mit einem Druckminderventil 42 ausgestattet ist. Das Druckminderventil 42 ist zwischen 0 und einem Maximaldruck M regelbar, wobei der Maximaldruck M so gewählt ist, daß er unterhalb eines Drucks verbleibt, der für die Darmspülung kritisch wäre. Zwischen diesen beiden Endstellungen ist das Druckminderventil 42 stufenlos einstellbar.

Die Ableitung 32 ist ferner mit einem an sich bekannten Konus 50 für die Verbindung mit dem künstlichen Darmausgang verbunden. Für die Befüllung der Spülflüssigkeitskammer 14 wird das Druckminderventil 52 in die Stellung 0 gebracht so daß die Ableitung 32 verschlossen ist. Nach ausreichender Befüllung wird das Sperrventil 48 geschlossen, die Pumpe 36 mit ihrem Druckstutzen in die Steckverbindung 40, die Beispielsweise als Bayonett-Steckverbindung ausgebildet sein kann, eingesetzt, und die Luftkammer 16 aufgepumpt. Sobald die Luftkammer 16 prall gefüllt ist, wird der Konus 54 in der gewünschten Weise appliziert und das Druckminderventil 52 auf den gewünschten Druck, einsprechend einer aufgetragenen Druckskala geöffnet. Die Spülflüssigkeit 14 wird dann über den Druck, der auf sie über die Membran 18 ausgeübt wird, aus der Spülflüssigkeitskammer 14 herausgedrückt. Nach dem Ende des Spülvorgangs wird die Spülflüssigkeitskammer 14 soweit kollabiert, daß die Membran 18 sich praktisch an die Außenwand 22 anlehnt, und dort ein minimales Spülflüssigkeitskammervolumen beläßt. Demgegenüber herrscht in der Luftkammer 16 weiter Überdruck, und die Luftkammer 16 hat ihr maximales Volumen erreicht.

Für die erneute Befüllung wird die Steckverbindung 40 geöffnet, so daß die in der Luftkammer 16 befindliche Luft ausströmen kann. Für die Aufbewahrung des Irrigators 10 wird nun die Restluft manuell aus der Luftkammer 16 herausgedrückt, so daß die Außenwand 20 sich konkav in die Außenwand 22 hinein wölbt. Dies kann gegebenenfalls durch ein Absaugen der Luft über den Sauganschluß der Pumpe 36 unterstützt werden. Der Irrigator 10 wird sorgfältigt gereinigt und kann auf ein kleines Packmaß zusammengefaltet werden.

Es versteht sich, daß verschiedene Abwandlungen der dargestellten erfindungsgemäßen Ausführungsform möglich sind, ohne die Grundgedanken der Erfindung zu verlassen. Beispielsweise kann die Steckverbindung 20 durch ein Ablaßventil ersetzt werden, das das Ablassen der Restluft ermöglicht. Ferner ist es möglich, die Zuleitung 30 und die Ableitung 32 an gegenüberliegenden Enden der Spülflüssigkeitskammer 14 anzuschließen. Die Zuleitung 30 kann auch mit einem regelrechten Trichter versehen sein, um die Zuleitung von Wasser zu erleichtern. Ferner kann auch die Zuleitung 30 mit der Ableitung 32 zusammengefaßt sein, wobei das Druckminderventil 52 dann über ein integriertes Rückschlagventil in Gegenrichtung überbrückt ist, das beim Wassereinlaß leicht öffnet. Hierzu ist dann in dem Konus 54 ein entsprechender Trichtervorgesehen, so daß eine Befüllung und der Betrieb des Irrigators 10 lediglich über einen Schlauch möglich ist.

## Patentansprüche

1. Irrigator, mit einem mit Spülflüssigkeit zu füllenden Beutel, der an einem Ableitungsschlauch angeschlossen ist, dadurch gekennzeichnet, daß der Beutel (12) eine verschließbare Spülflüssigkeitskammer (14) aufweist, die durch eine Membran (18) von einer Luftkammer (16) getrennt ist, die über eine Pumpe (36) unter Druck setzbar ist.

2. Irrigator nach Anspruch 1, dadurch gekennzeichnet, daß die Pumpe (36), insbesondere eine Ball-Handpumpe, über einen Schlauch (38) an die Luftkammer (16) des Beutels (12) angeschlossen ist.

3. Irrigator nach Anspruch 1 oder 2, dadurch gekennzeichnet, daß die Luftkammer (16), der Schlauch (38) oder die Pumpe (36) ein Ablaßventil aufweisen, mit welchem der Druck in der Luftkammer (16) reduzierbar ist.

4. Irrigator nach einem der vorhergehenden Ansprüche, dadurch gekennzeichnet, daß der Ableitungsschlauch (32) ein Ventil (52) für die Spülflüssigkeit aufweist.

5. Irrigator nach Anspruch 4, dadurch gekennzeichnet, daß das Ventil als Druckminderventil (52) ausgebildet ist, dessen maximaler Abgabedruck voreingestellt ist, und dessen Abgabedruck zwischen null und dem voreingestellten Maximalabgabedruck regelbar ist.

6. Irrigator nach einem der vorhergehenden Ansprüche, dadurch gekennzeichnet, daß an die Spülflüssigkeitskammer (14) ein Zuleitungsschlauch (30) angeschlossen ist, der einen Verschluß (Sperrventil 48) aufweist.

7. Irrigator nach einem der vorhergehenden Ansprüche, dadurch gekennzeichnet, daß der Zuleitungsschlauch (30) und der Ableitungsschlauch (32) gemeinsam in die Spülflüssigkeitskammer (14) geführt sind und insbesondere über ein T-Stück (46) miteinander verbunden sind.

8. Irrigator nach einem der vorhergehenden Ansprüche, dadurch gekennzeichnet, daß die Membran (18) zwischen einer ersten Endstellung, in welcher das Spülflüssigkeitskammervolumen minimal und das Luftkammervolumen maximal ist und einer zweiten Endstellung, in welcher das Spülflüssigkeitskammervolumen maximal und das Luftkammervolumen minimal ist, beweglich ist, wobei die maximalen Kammervolumina im wesentlichen gleich und insbesondere die minimalen Kammervolumina im wesentlichen gleich sind.

9. Irrigator nach einem der vorhergehenden Ansprüche, dadurch gekennzeichnet, daß als Beutelmaterial ein flexibles, im wesentlichen jedoch nicht elastisch dehnbares Material wie Polyethylen oder Polyvinylchlorid eingesetzt ist und der Beutel (12) seitlich mindestens je eine Volumenvergrösserungsfalte aufweist.

10. Irrigator nach einem der vorhergehenden Ansprüche, dadurch gekennzeichnet, daß der Beutel (12) spülflüssigkeitskammerseitig einen Füllhöhenanzeiger (Markierungen 44) aufweist und insbesondere transparent ist.
